(19) Europäisches Patentamt
European Patent Office
Office européen des brevets

(11) EP 3 052 929 B1

(12) **EUROPEAN PATENT SPECIFICATION**

(45) Date of publication and mention
of the grant of the patent:
**09.09.2020 Bulletin 2020/37**

(21) Application number: **14744924.3**

(22) Date of filing: **24.07.2014**

(51) Int Cl.:
***G01N 27/407*** (2006.01)

(86) International application number:
**PCT/GB2014/052267**

(87) International publication number:
**WO 2015/011480 (29.01.2015 Gazette 2015/04)**

(54) **METHOD AND APPARATUS FOR SENSING MOLECULAR GASES**

VERFAHREN UND VORRICHTUNG ZUR ERFASSUNG MOLEKULARER GASE

PROCÉDÉ ET APPAREIL POUR DÉTECTER DES GAZ MOLÉCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2013 GB 201313396**

(43) Date of publication of application:
**10.08.2016 Bulletin 2016/32**

(73) Proprietors:
• **Cambridge Enterprise Limited
Cambridge
Cambridgeshire CB2 1TN (GB)**
• **Environmental Monitoring And Control Limited
Tollgate Industrial Estate
Stafford
Staffordshire ST16 3SU (GB)**

(72) Inventors:
• **HILLS, Matthew Paul
Stafford
Staffordshire ST16 3SU (GB)**
• **SCHWANDT, Carsten
Cambridge, CB3 0GT (GB)**
• **KUMAR, Vasant
Cambridge, CB3 0GT (GB)**

(74) Representative: **Goodman, Simon John Nye
Reddie & Grose LLP
The White Chapel Building
10 Whitechapel High Street
London E1 8QS (GB)**

(56) References cited:
**EP-A1- 0 468 249        JP-A- S 603 547
US-A- 5 080 775         US-A- 5 954 930
US-B1- 6 179 992**

**Description**

Field of Invention

The invention relates to a method and an apparatus for measuring the pressure or concentration of molecular gases, using a sensor that comprises a combination of solid ion-conductors in conjunction with a gas-sensitive measurement electrode, or auxiliary phase, and a reference electrode.

Background

The monitoring and control of the pressures or concentrations of molecular gases such as carbon oxides ($CO_2$, CO), sulphur oxides ($SO_3$, $SO_2$), nitrogen oxides ($NO_2$, NO), chlorine ($Cl_2$) and others is an important scientific and technological issue. There are a plethora of possible applications in the realms of environmental monitoring and process control. Examples include the measurement of $CO_2$ gas in the ambient atmosphere in the context of global warming, the measurement of $CO_2$ gas in buildings and cars in the context of on-demand ventilation control, and the measurement of various toxic gases in the context of monitoring combustion or metallurgical processes.

At present, the quantitative analysis of the gases mentioned is not straightforward. For quantitative $CO_2$ analysis, the predominantly applied method is infra-red spectroscopy. This requires bulky, sophisticated and expensive equipment and makes on-line analysis cumbersome. Even though very modern devices are now less bulky, they still cannot be miniaturised down to the size of typical electrochemical sensors. For quantitative $SO_3$ analysis, the only reliable method is wet chemical analysis. This involves complex and lengthy procedures and makes rapid on-line monitoring impossible. Spectroscopic methods can also be used but the additional complication is that the determination of small quantities of $SO_3$ in the presence of large quantities of $SO_2$ is critically affected by the similar absorption behaviours of both gases, rendering selective detection difficult. Problems of similar kinds are also encountered in the analysis of the other gases mentioned.

There is a pressing need to develop gas sensing systems for environmental monitoring and process control that should preferably be accurate and reliable, small and light, flexible to install and easy to use, as well as robust and affordable. These systems should also possess high sensitivity and selectivity as well as long-term stability and drift-free performance.

Attempts have been made to develop potentiometric sensors based on solid ion-conductors (solid electrolytes) to meet the requirements of a practical, commercial, gas-sensing system.

Sensors relying on solid ion-conductors with oxide ion or proton conductivity have become established technologies for the sensing of oxygen and hydrogen, respectively. Sensors using solid ion-conductors with metal ion conductivity are also known, with the majority of research efforts directed towards the sensing of $CO_2$ and $SO_3$, but no commercial breakthrough has as yet been achieved because all such sensors for molecular gases have failed to deliver adequate performance.

There are a large number of research articles on the subject of chemical sensors based on solid ion-conductors for use for sensing gases. Recent thorough and detailed review articles that define the state-of-the-art in the field are the following:

- H.-H. Möbius, Galvanic solid electrolyte cells for the measurement of CO2 concentrations, Journal of Solid State Electrochemistry 8 (2004) pp. 94-109.
- J.W. Fergus, A review of electrolyte and electrode materials for high temperature electrochemical CO2 and SO2 gas sensors, Sensors and Actuators B 134 (2008) pp. 1034-1041.
- P. Pasierb, M. Rękaskas, Solid-state potentiometric gas sensors - current status and future trends, Journal of Solid State Electrochemistry 13 (2009) pp. 3-25.

It is noted that the terms 'pressure' and 'concentration' are used interchangeably in relation to measurand gases throughout this document. Their conversion is possible via the ideal gas law $p = (n\,R\,T)\,/\,V = c\,R\,T$, where p is pressure, n is number of moles, R is the universal gas constant, T is absolute temperature, V is volume, and c is concentration.

Potentiometric sensors

The potentiometric (or electrolytic, or galvanic) sensors described in the prior art for the sensing of gases such as $CO_2$, $SO_3$, $NO_2$ and $Cl_2$ using solid ion-conductors are based on the same fundamental sensor concept which comprises three distinct components: a measurement electrode and a reference electrode electrically connected through a solid ion-conductor (solid electrolyte).

More specifically, a typical solid-state potentiometric sensor used for the sensing of molecular gases comprises a gas-sensitive measurement electrode (or measuring electrode, or auxiliary phase, or auxiliary electrode) that equilibrates chemically with a target molecular-gas species in a measurand gas, a solid ion-conductor (solid electrolyte) that contains a mobile metal ion, and a reference electrode that provides a known, or predetermined, reference potential. The cell potential (cell voltage) is the difference between the electrode potential of the measurement electrode (or auxiliary phase) and the electrode potential of the reference electrode.

Solid ion-conductors

Solid ion-conductors (or solid electrolytes) are solid materials that possess a significant amount of mobile ions that render the materials ionically conducting. Solid ion-conductors may be divided into salt ion-conductors and ceramic ion-conductors. Also known are glass ion-conductors, but their properties are largely similar to those of the ceramic type and are not considered separately in the present context. Where appropriate in this document, for brevity, the term ceramic ion-conductor is used to include both ceramic and glass ion-conductors.

Salt solid ion-conductors have been known for over one hundred years and include, but are not limited to, materials such as sodium carbonate ($Na_2CO_3$), sodium nitrate ($NaNO_3$), sodium fluoride ($NaF$), sodium meta-silicate ($Na_2SiO_3$), sodium ortho-silicate ($Na_4SiO_4$), sodium phosphate ($Na_3PO_4$), sodium sulphate ($Na_2SO_4$), sodium chloride ($NaCl$), the analogous lithium (Li), potassium (K), rubidium (Rb), and caesium (Cs), compounds, as well as a number of analogous alkaline earth metal compounds, most importantly, calcium fluoride ($CaF_2$). Many more salt ion-conductors have been named in the literature and are known to those skilled in the art. The mobile ion in salt ion-conductors may be a metal ion (as in, for example, $Na_2CO_3$ $Na_2SO_4$, $Na_2SiO_3$) or a non-metal ion (as in, for example, $CaF_2$). This class of ion-conductor may be termed a salt ion-conductor or salt electrolyte even though a particular ion-conductor may be composite material, with the salt ion-conductor supported in or by a matrix such as a ceramic matrix.

Ceramic solid ion-conductors have been known for several decades and include, but are not limited to, materials such as fully or partially stabilised zirconia, partially substituted ceria, partially substituted perovskites, Li-β-alumina, Na-β-alumina, K-β-alumina, Mg-β-alumina, Ca-β-alumina, Sr-β-alumina, Ba-β-alumina, Cu-β-alumina, Ag-β-alumina, rare earth metal β-aluminas, NASICON, LISICON, CUSICON, and various oxide-based and fluoride-based glasses. Many more ceramic ion-conductors have been named in the literature and are known to those skilled in the art.

The ceramic solid ion-conductors with metal ion conductivity that are most widely applied are Na-β-alumina and NASICON. Na-β-alumina is a double oxide of $Na_2O$ and $Al_2O_3$ with the general stoichiometry of $Na_2O \cdot xAl_2O_3$ (x>1) and optional additions of stabilising oxides such as $Li_2O$ or $MgO$. It is a two-dimensional ion-conductor, in which spinel blocks are bridged via oxygen atoms and the layers between the spinel blocks host the $Na^+$ ions and allow for their fast transport. Specific compositions in this class of material are $NaAl_5O_8$ (Na-β"-alumina) and $NaAl_{11}O_{17}$ (Na-β-alumina). In the present context, the term 'Na-β-alumina' shall comprise any oxide from the Na-Al-O systems, including materials of the specific compositions above, materials of compositions deviating from the precise compositions above, mixtures of two or more materials of the above or deviating compositions, and materials with and without additional stabilising oxides. NASICON is a solid solution of $Na_4Zr_2(SiO_4)_3$ and $NaZr_2(PO_4)_3$ with the general stoichiometry of $Na_{1+x}Zr_2Si_xP_{3-x}O_{12}$ (0<x<3). It is a three-dimensional ion-conductor, in which a large number of energetically similar filled and unfilled lattice sites exist that allow for fast $Na^+$ ion transport. A specific composition with high $Na^+$ ion conductivity is $Na_3Zr_2Si_2PO_{12}$.

This class of material may be termed ceramic ion-conductors or ceramic electrolytes.

In the following, a $Na^+$ ion-conductor is used to exemplify the fundamental concept of gas sensing by means of solid metal-ion-conductors, but it is understood that analogous approaches are possible with other solid metal-ion-conductors.

Gas-sensitive measurement electrodes

A gas-sensitive measurement electrode, or auxiliary electrode or auxiliary phase, comprises a material that is able to equilibrate chemically with a target species in an adjacent gas, and in particular a target species in a measurand gas comprising a mixture of at least the target gas species and another gas or gases, which may be termed a carrier gas or balance gas. The measurement electrode is in direct contact with a solid metal-ion-conductor and typically, but not necessarily, contains at least one compound of the metal whose ion is mobile in the solid metal-ion-conductor. Through this equilibration the chemical activity of this metal in the measurement electrode becomes uniquely defined. Four examples will be elaborated in greater detail as follows, for measuring $CO_2$, $SO_3$, $NO_2$ and $Cl_2$. Other systems are known to those skilled in the art.

The equilibration of $Na_2CO_3$ as the measurement electrode with $CO_2$ as the target species in the gas occurs via the following chemical equilibrium:

$$Na_2CO_3 = 2\ Na + CO_2(g) + 0.5\ O_2(g) \qquad (1)$$

This equilibrium reaction fixes the chemical activity of Na in the $Na_2CO_3$. It is noted that not only $CO_2$ but also $O_2$ has an influence on the chemical activity of Na. Typically, in order to measure $CO_2$ concentration or pressure, the concentration or pressure of $O_2$ is measured separately by means of an $O_2$ sensor, so that the influence due to $CO_2$ can readily be singled out.

To measure $SO_3$ concentration or pressure, the equilibration of $Na_2SO_4$ as the measurement electrode with $SO_3$ as the target species in an adjacent or surrounding gas occurs via the following chemical equilibrium:

$$Na_2SO_4 = 2\ Na + SO_3(g) + 0.5\ O_2(g) \qquad (2)$$

This equilibrium reaction fixes the chemical activity of Na in the $Na_2SO_4$.

The equilibration of $NaNO_3$ as the measurement electrode with $NO_2$ as the target species in an adjacent or surrounding gas occurs via the following chemical equilibrium:

$$NaNO_3 = Na + NO_2(g) + 0.5\ O_2(g) \qquad (3)$$

This equilibrium reaction fixes the chemical activity of Na in the $NaNO_3$.

The equilibration of NaCl as the measurement electrode with $Cl_2$ as the target species in an adjacent or surrounding gas occurs via the following chemical equilibrium:

$$NaCl = Na + 0.5\ Cl_2(g) \qquad (4)$$

This equilibrium reaction fixes the chemical activity of Na in the NaCl.

In all of the above cases, the Na-containing compound in the measurement electrode (or auxiliary phase) equilibrates with the target species in the gas, which fixes the chemical activity of the Na in the measurement electrode. The Na in the $Na^+$ ion-conductor in contact with the measurement electrode then equilibrates with the Na in the measurement electrode. This determines the electrode potential of the measurement electrode. Other measurement electrode materials may be used and analogous equilibria can be written with such compounds containing a different metal, and the ion corresponding to this different metal should then preferably be the mobile ion in the solid ion-conductor.

Reference electrodes

A reference electrode is a material that provides a known, or predetermined, chemical activity of one of the elements involved in the sensor chemistry. The reference electrode is in direct contact (electrical contact) with a solid metal-ion-conductor and typically, but not necessarily, contains the metal, or at least one compound of the metal, whose ion is mobile in the solid metal-ion-conductor. Through the composition of the reference electrode the chemical activity of this metal in the reference electrode becomes uniquely defined.

Reference electrodes for metallic elements can be of various forms. The simplest one is the pure metal, protected from the gas (i.e. the measurand gas containing the target compound) by a hermetic seal. More complex ones are mixtures of compounds of which at least one contains the metal, and these are either protected from the gas by a seal or open to the gas. In the former case, species of the gas do not partake in the reactions in the reference electrode, while in the latter case they may do so. In all cases the compounds and phases in the reference electrode should preferably be chosen such that the chemical activity of the metal in the reference electrode becomes uniquely defined. Several examples will be elaborated in greater detail in the following, while many other systems are known to those skilled in the art.

For a Na reference electrode, the following systems have been described in the prior art: pure Na protected from the gas by means of a seal; a mixture of the two condensed phases Au and $Au_2Na$ protected from the gas by means of a seal; a mixture of three condensed phases of the Na-Co-O or Na-Ni-O ternary systems protected from the gas by means of a seal; a mixture of two condensed phases and $O_2$ from the gas using systems such as $Na_2ZrO_3$, $ZrO_2$, $O_2$; $Na_2MoO_4$, $MoO_3$, $O_2$; $Na_2WO_4$, $WO_3$, $O_2$; $Na_2SnO_3$, $SnO_2$, $O_2$; $Na_2Ti_6O_{13}$, $TiO_2$, $O_2$; $ZrSiO_4$, $ZrP_2O_7$, $O_2$; $Na_2Si_2O_5$, $SiO_2$, $O_2$; $Na_2Ti_6O_{13}$, $Na_2Ti_3O_7$, $O_2$; $Na_2Si_2O_5$, $SiO_2$, $O_2$; $Na_2Si_2O_5$, $Na_2SiO_3$, $O_2$; $Na_2Ge_4O_9$, $GeO_2$, $O_2$.

When pure Na is used as the reference electrode, the chemical activity of Na in the reference electrode is unity. When multinary systems containing Na are used, the chemical activity of Na is fixed at values lower than unity. The Na in the $Na^+$ ion-conductor in contact with the reference electrode equilibrates with the Na in the reference electrode. This determines the electrode potential of the reference electrode. It is understood that analogous equilibria can be written with compounds containing a different metal, and that the ion corresponding to this different metal should then preferably be the mobile ion in the solid ion-conductor.

Cell potential

In order to measure the cell potential of a potentiometric sensor, the measurement electrode and the reference electrode are contacted with electronic conductors, typically of a noble metal, and the electronic conductors are connected to the two terminals of a voltmeter.

The cell potential arises from the difference in chemical activity, at the measurement electrode and at the reference electrode, of the element whose ion is mobile in the solid ion-conductor. The cell potential is given by the well-known Nernst equation:

$$U = -\frac{RT}{zF}\ln\frac{a''}{a'} \tag{5}$$

where $U$ is the cell potential, $R$ is the universal gas constant of 8.3145 J mol$^{-1}$ K$^{-1}$, $z$ is the number of elemental charges on the mobile ion (counted positive for cation-conductors and negative for anionconductors), $F$ is the Faraday constant of 96487 C mol$^{-1}$, $a''$ is the chemical activity at the measurement electrode of the element whose ion is mobile in the solid ion-conductor, and a' is the chemical activity at the reference electrode of this element.

For a known temperature and a known chemical activity a' at the reference electrode, the measured cell potential is a direct function of the unknown chemical activity $a''$ at the measurement electrode. The chemical activity $a''$ in turn is a direct function of the pressure or concentration of the target species in the gas, consequent of the equilibration reactions (1) to (4). In this way the pressure or concentration of the target species is experimentally accessible.

In case the solid ion-conductor is a Na$^+$ ion-conductor, the expression for the cell potential is:

$$U = -\frac{RT}{F}\ln\frac{a''_{Na}}{a'_{Na}} \tag{6}$$

where $a''_{Na}$ is the chemical activity of Na at the measurement electrode and $a'_{Na}$ is the chemical activity of Na at the reference electrode.

Development of sensors

Historically, the first solid state potentiometric sensors for the sensing of gases such as $CO_2$ and $SO_3$ involved salt solid metal-ion-conductors such as $Na_2CO_3$ and $Na_2SO_4$. As salt bodies are not mechanically robust and cannot be machined into complex shapes, the early sensor designs were assembled around salt discs and were operated as gas-concentration cells with different pressures or concentrations of the target species on both sides. However, this research line was soon abandoned as it became clear that no practically-useful devices could be constructed. Research then regained momentum with the advent of ceramic solid metal-ion-conductors. As these materials can be sintered and machined into diverse mechanically-stable shapes, sensor designs became more flexible and sensor assembly became simpler.

One possible potentiometric sensor for the combined sensing of $CO_2$ and $O_2$ gases comprises $Na_2CO_3$ as the gas sensitive measurement electrode, Na-β-alumina as the solid metal-ion-conductor, and Na of known chemical activity as the reference electrode. The galvanic cell symbol of this type of sensor is:

$$CO_2,\ O_2,\ Na_2CO_3|\ \text{Na-β-alumina} \mid \text{Na} \tag{7}$$

where the reaction at the measurement electrode is:

$$Na_2CO_3 = 2\ Na + CO_2(g) + 0.5\ O_2(g) \tag{8}$$

The chemical activity of Na at the measurement electrode $a''_{Na}$ is given through the chemical equilibration of the $Na_2CO_3$ of the measurement electrode with the $CO_2$ and $O_2$ in the gas. This activity can readily be shown to be:

$$a''_{Na} = \frac{a_{Na_2CO_3}^{1/2}}{p_{CO_2}^{1/2}\, p_{O_2}^{1/4}}\exp\frac{\Delta G_{Na_2CO_3}^0 - \Delta G_{CO_2}^0}{2RT} \tag{9}$$

5

where $p_{CO2}$ is the pressure of $CO_2$ in atm in the gas, $p_{O2}$ is the pressure of $O_2$ in atm in the gas, $a_{Na2CO3}$ is the chemical activity of $Na_2CO_3$ at the measurement electrode, and the $\Delta G^0$ values are the standard Gibbs free energies of formation of the compounds indicated.

The corresponding cell potential $U$ is:

$$U = U^0 - \frac{RT}{F}\ln\frac{a_{Na_2CO_3}^{1/2}}{p_{CO_2}^{1/2}p_{O_2}^{1/4}a'_{Na}} \tag{10}$$

where $U^0$ is the known and constant standard electrode potential and $a'_{Na}$ is the chemical activity of Na at the measurement electrode.

The cell potential can be simplified to:

$$U = U^0 - \frac{RT}{F}\ln\frac{1}{p_{CO_2}^{1/2}p_{O_2}^{1/4}} \tag{11}$$

in the case where pure $Na_2CO_3$ is used at the measurement electrode and pure Na is used at the reference electrode, so both these activities are unity.

The cell potential $U$ depends on the pressures or concentrations of $CO_2$ and $O_2$ in the gas adjacent to the measurement electrode and the temperature. If $p_{O2}$ is known or measured separately, and if the temperature is known, then $p_{CO2}$ becomes a measurable quantity. It is understood that this sensing method rests on the assumption that the cell potential established is the one predicted by the Nernst equation.

One possible potentiometric sensor for the combined sensing of $SO_3$ and $O_2$ gases comprises $Na_2SO_4$ as the gas sensitive measurement electrode, Na-β-alumina as the solid metal-ion-conductor, and Na of known chemical activity as the reference electrode. The galvanic cell symbol of this type of sensor is:

$$SO_3,\ O_2,\ Na_2SO_4|\ \text{Na-β-alumina}\ |\ Na \tag{12}$$

where the reaction at the measurement electrode is:

$$Na_2SO_4 = 2\ Na + SO_3(g) + 0.5\ O_2(g) \tag{13}$$

The chemical activity of Na at the measurement electrode $a''_{Na}$ is given through the chemical equilibration of the $Na_2SO_4$ of the measurement electrode with the $SO_3$ and $O_2$ in the gas. This activity can readily be shown to be:

$$a''_{Na} = \frac{a_{Na_2SO_4}^{1/2}}{p_{SO_3}^{1/2}p_{O_2}^{1/4}}\exp\frac{\Delta G_{Na_2SO_4}^0 - \Delta G_{SO_3}^0}{2RT} \tag{14}$$

where $p_{SO3}$ is the pressure of $SO_3$ in atm in the gas, $p_{O2}$ is the pressure of $O_2$ in atm in the gas, $a_{Na2SO4}$ is the chemical activity of $Na_2SO_4$ at the measurement electrode, and the $\Delta G^0$ values are the standard Gibbs free energies of formation of the compounds indicated.

The corresponding cell potential $U$ is:

$$U = U^0 - \frac{RT}{F}\ln\frac{a_{Na_2SO_4}^{1/2}}{p_{SO_3}^{1/2}p_{O_2}^{1/4}a'_{Na}} \tag{15}$$

where $U^0$ is the known and constant standard electrode potential and $a'_{Na}$ is the chemical activity of Na at the measurement electrode.

The cell potential can be simplified to:

$$U = U^0 - \frac{RT}{F} \ln \frac{1}{p_{SO_3}^{1/2} p_{O_2}^{1/4}} \qquad (16)$$

in case pure $Na_2SO_4$ is used at the measurement electrode and pure Na is used at the reference electrode, so both these activities are unity.

The cell potential $U$ depends on the pressures or concentrations of $SO_3$ and $O_2$ in the gas adjacent to the measurement electrode and the temperature. If $p_{O_2}$ is known or measured separately, and if the temperature is known, then $p_{SO_3}$ becomes a measurable quantity. It is understood that this sensing method rests on the assumption that the cell potential established is the one predicted by the Nernst equation.

One possible potentiometric sensor for the combined sensing of $NO_2$ and $O_2$ gases comprises $NaNO_3$ as the gas sensitive measurement electrode, Na-β-alumina as the solid metal-ion-conductor, and Na of known chemical activity as the reference electrode. The galvanic cell symbol of this type of sensor is:

$$NO_2, O_2, NaNO_3 \mid Na\text{-}\beta\text{-alumina} \mid Na \qquad (17)$$

where the reaction at the measurement electrode is:

$$NaNO_3 = Na + NO_2(g) + 0.5\ O_2(g) \qquad (18)$$

The chemical activity of Na at the measurement electrode $a''_{Na}$ is given through the chemical equilibration of the $NaNO_3$ of the measurement electrode with the $NO_2$ and $O_2$ in the gas. This activity can readily be shown to be:

$$a''_{Na} = \frac{a_{NaNO_3}}{p_{NO_2} p_{O_2}^{1/2}} \exp \frac{\Delta G^0_{NaNO_3} - \Delta G^0_{NO_2}}{RT} \qquad (19)$$

where $p_{NO2}$ is the pressure of $NO_2$ in atm in the gas, $p_{O2}$ is the pressure of $O_2$ in atm in the gas, $a_{NaNO3}$ is the chemical activity of $NaNO_3$ at the measurement electrode, and the $\Delta G^0$ values are the standard Gibbs free energies of formation of the compounds indicated.

The corresponding cell potential $U$ is:

$$U = U^0 - \frac{RT}{F} \ln \frac{a_{NaNO_3}}{p_{NO_2} p_{O_2}^{1/2} a'_{Na}} \qquad (20)$$

where $U^0$ is the known and constant standard electrode potential and $a'_{Na}$ is the chemical activity of Na at the measurement electrode.

The cell potential can be simplified to:

$$U = U^0 - \frac{RT}{F} \ln \frac{1}{p_{NO_2} p_{O_2}^{1/2}} \qquad (21)$$

in case pure $NaNO_3$ is used at the measurement electrode and pure Na is used at the reference electrode, so both these activities are unity.

The cell potential $U$ depends on the pressures or concentrations of $NO_2$ and $O_2$ in the measurement electrode adjacent to the gas and the temperature. If $p_{O2}$ is known or measured separately, and if the temperature is known, then $p_{NO2}$ becomes a measurable quantity. It is understood that this sensing method rests on the assumption that the cell potential established is the one predicted by the Nernst equation.

One possible potentiometric sensor for the sensing of $Cl_2$ gas comprises NaCl as the gas sensitive measurement electrode, Na-β-alumina as the solid metal-ion-conductor, and Na of known chemical activity as the reference electrode. The galvanic cell symbol of this type of sensor is:

$$\text{Cl}_2, \text{NaCl} \mid \text{Na-}\beta\text{-alumina} \mid \text{Na} \qquad (22)$$

where the reaction at the measurement electrode is:

$$\text{NaCl} = \text{Na} + 0.5\ \text{Cl}_2(g) \qquad (23)$$

The chemical activity of Na at the measurement electrode $a''_{Na}$ is given through the chemical equilibration of the NaCl of the measurement electrode with the $\text{Cl}_2$ in the gas. This activity can readily be shown to be:

$$a''_{Na} = \frac{a_{NaCl}}{p_{Cl_2}^{1/2}} \exp \frac{\Delta G^0_{NaCl}}{RT} \qquad (24)$$

where $p_{Cl2}$ is the pressure of $\text{Cl}_2$ in atm in the gas, $a_{NaCl}$ is the chemical activity of NaCl at the measurement electrode, and the $\Delta G^0$ value is the standard Gibbs free energy of formation of the compound indicated.

The corresponding cell potential $U$ is:

$$U = U^0 - \frac{RT}{F} \ln \frac{a_{NaCl}}{p_{Cl_2}^{1/2} a'_{Na}} \qquad (25)$$

where $U^0$ is the known and constant standard electrode potential and $a'_{Na}$ is the chemical activity of Na at the measurement electrode.

The cell potential can be simplified to:

$$U = U^0 - \frac{RT}{F} \ln \frac{1}{p_{Cl_2}^{1/2}} \qquad (26)$$

in case pure NaCl is used at the measurement electrode and pure Na is used at the reference electrode, so both these activities are unity.

The cell potential $U$ depends on the pressure or concentration of $\text{Cl}_2$ in the gas adjacent to the measurement electrode and the temperature. If the temperature is known, then $p_{Cl2}$ becomes a measurable quantity. It is understood that this sensing method rests on the assumption that the cell potential established is the one predicted by the Nernst equation.

The standard Gibbs free energy values $\Delta G^0$ required in the formulae above are available from tables and databases known to those skilled in the art, for example, JANAF Tables, Barin Knacke, or HSC Chemistry for Windows.

Prior art

Patent publication US5,954,930 describes a gas sensor in which a reference electrode is provided by a glass phase in contact with an ion-conducting solid electrolyte, in which the glass phase has dissolved therein the species for which the solid electrolyte is conductive. A variety of ceramic materials are proposed for the solid electrolyte.

Patent publication US 5,080,775 describes a gas detector which has a memory function for detected data. The sensor comprises laminated layers having a detecting electrode, a first conductive solid electrolyte, a polarisation electrode, a second conductive solid electrolyte, and a reference electrode.

Patent publication JP60003547 describes a gas sensor comprising a solid electrolyte which comprises a sodium constituent and a reference electrode made of sodium or a sodium alloy.

Problems

It should be noted that, despite the straightforward scientific concepts described above, the substantial volume of literature on the subject, and the huge demand for monitoring and control systems for molecular gases, no sensor of the above type has as yet proven to be viable in a practical application. The major problems observed by developers and users of such sensors are the following: the cell potential does not reach the thermodynamically-expected value according to the Nernst equation, nominally-identical sensors yield different cell potentials under the same experimental conditions, and the cell potential drifts with time.

Summary of Invention

The present invention provides a method and apparatus for measuring, or for quantitatively sensing, the pressure or concentration of a molecular gas, such as $CO_2$, $SO_3$, $NO_2$ or $Cl_2$, as defined in the appended independent claims to which reference should now be made. Preferred or advantageous features of the invention are set out in dependent subclaims.

A first preferred embodiment of the invention may thus provide an apparatus comprising: a ceramic or glass, solid ion-conductor (or solid electrolyte) that contains a first metal ion as its mobile species; a salt ion-conductor (or solid electrolyte) in electrical contact with the ceramic, or glass, ion-conductor and that contains the first or a second, different, ion as its mobile species; a gas-sensitive measurement electrode in electrical contact with one of the ion conductors, the measurement electrode including an auxiliary phase comprising one or more chemical compounds that is or are able to equilibrate chemically with a target molecular species in a gas, and that contains at least one compound of the metal whose ion is mobile in the ion-conductor; and a reference electrode that provides a predetermined, or reference, chemical activity of the metal whose ion is mobile in the other ion-conductor and that is in electrical contact with the other ion-conductor. In this structure, the ceramic, or glass, ion-conductor and the salt ion-conductor form an electrolyte of the sensor between the electrodes.

A further preferred embodiment of the invention may thus provide an apparatus comprising: a first ion-conductor which is a ceramic or glass ion-conductor; a second ion-conductor which is a salt ion-conductor; a third ion-conductor which is a ceramic or glass ion-conductor; a gas-sensitive measurement electrode; and a reference electrode. The first, second and third ion-conductors contain first, second and third metal ions as their mobile species, and any of the first, second and third ion species may be the same as or different from each other. The second ion-conductor separates, and is in electrical contact with, the first and the third ion-conductors. The measurement electrode contains an auxiliary phase that comprises one or more chemical compounds that is or are able to equilibrate chemically with a molecular species in a gas (to which the measurement electrode is exposed during use of the sensor) and that contains at least one compound of the metal whose ion is mobile in the first ion-conductor. The measurement electrode is in electrical contact with the first ion-conductor. The reference electrode provides a predetermined, or reference, chemical activity of the metal whose ion is mobile in the third ion-conductor and is in electrical contact with the third ion-conductor. The second ion-conductor separates, and is in electrical contact with, the first and the third ion-conductors. In this structure, the ceramic, or glass, ion-conductors and the salt ion-conductor form an electrolyte of the sensor between the electrodes.

An apparatus of this type may advantageously be able to establish a thermodynamically-expected cell potential and/or not be subject to potential drift.

In other words, embodiments of the invention may advantageously provide a method and apparatus for the quantitative sensing of molecular gases. The apparatus may comprise a gas-sensitive measurement electrode, an electrically-connected series of solid ion-conductors including at least a salt ion-conductor and a ceramic or glass ion-conductor, and a reference electrode. The cell potential generated may then be a direct function of the pressure or concentration of the molecular gas to be sensed.

The inventors' current understanding is that the reason for the unsatisfactory performance of the sensors described in the prior art, which has not been appreciated in the prior art, lies in the occurrence of partial electronic conduction through the ceramic solid ion-conductors. This phenomenon causes cell potentials lower than theoretically-expected values. Electronic conduction across an ion-conductor between two electrodes may occur when the chemical activity of the element whose ion is mobile in the ion-conductor is outside the electrolytic domain of the ion-conductor at either or both electrodes, so that the material may no longer be considered a pure ion-conductor but must instead be considered a mixed ionic and electronic conductor. The precise ratio of ionic conductivity to electronic conductivity may depend on various factors related to the properties of the solid conductor, the composition of the surrounding gas and the cell (or sensor) geometry, and may vary between individual experimental arrangements. This understanding accounts readily for the generally-observed lack of consistency and reproducibility described in the prior art.

The inventors have surprisingly found that the problem of experimentally-measured cell potentials being lower than the theoretically-expected ones may be overcome by a novel and unique modification of the galvanic cells employed. This modification consists in arranging in series a ceramic solid ion-conductor and a salt ion-conductor, or a first ceramic solid metal-ion-conductor, a salt solid ion-conductor, and a second ceramic solid metal-ion-conductor, and placing this arrangement in between a gas-sensitive measurement electrode and a reference electrode. The measurement electrode and/or the reference electrode may be conventional electrodes, from conventional galvanic cell systems.

Such an arrangement has been found in the inventors' experiments to establish the theoretically-expected cell potentials and to show no cell potential drift.

In a preferred embodiment of the invention, two ceramic solid metal-ion-conductors are physically separated by a salt solid ion-conductor. This arrangement produces good performance and is advantageously straightforward to manufacture and to package.

Without the invention being limited to the following theory, the inventors believe that the salt solid ion-conductor changes the transference properties of the ionic and electronic charge carriers across the galvanic cell. This occurs in such a way that the ionic conductivity in each cell component remains sufficient to enable the build-up of a measurable cell potential, but the electronic conductivity across the entire cell is diminished to such an extent that deviations from the theoretical cell potential become small or negligible.

To achieve more rapid measurements, the physical dimensions of a sensor embodying the invention may be made a small as possible, and in particular the thickness of the salt ion-conductor, as well as the thicknesses of the ceramic ion-conductors, may be made as small as possible. In practice, the inventors have found that the thickness of the salt ion-conductor is of particular importance. In addition, to increase the speed of measurement, the temperature of the sensor may be raised. Advantageously, a minimum operating temperature of the sensor may be 300°C, but more rapid measurement may be achieved at temperatures above 450°C. If measurements are being made in ambient temperatures within these ranges, then rapid measurements may be achieved. If measurements are to be made in lower ambient temperatures, then a sensor may be heated, for example electrically heated, while measurements are made. Sensors embodying the invention may advantageously be small in size, so that little energy is required to heat a sensor to a desired measuring temperature range.

Specific Embodiments

Specific embodiments of the invention will now be described by way of example, with reference to the accompanying drawings, in which:

Figure 1 is a schematic cross-section of a first embodiment of the invention in the form of a galvanic cell for the quantitative sensing of molecular gases;

Figure 2 is a schematic cross-section of a second embodiment of the invention in the form of a galvanic cell for the sensing of molecular gases;

Figure 3 is a schematic cross-section of a third embodiment of the invention in the form of a galvanic cell for the sensing of molecular gases;

Figure 4 is a schematic cross-section of a fourth embodiment of the invention in the form of a galvanic cell for the sensing of $CO_2$ and $O_2$ gases;

Figure 5 is a graph showing the cell potential of the cell in Figure 4 as a function of the $CO_2$ concentration of a measured gas;

Figure 6 is a graph showing the cell potential of the cell in Figure 4 as a function of the $O_2$ concentration of the measured gas;

Figure 7 is a graph showing the time-dependent cell potential of the cell in Figure 4 when responding to a variation of the $CO_2$ and $O_2$ concentrations of the measured gas;

Figure 8 is a schematic cross-section of a fifth embodiment of the invention in the form of a galvanic cell for the sensing of $SO_3$ and $O_2$ gases; and

Figure 9 is a graph showing the cell potential of the cell in Figure 8 as a function of the $SO_3$ concentration of the measured gas.

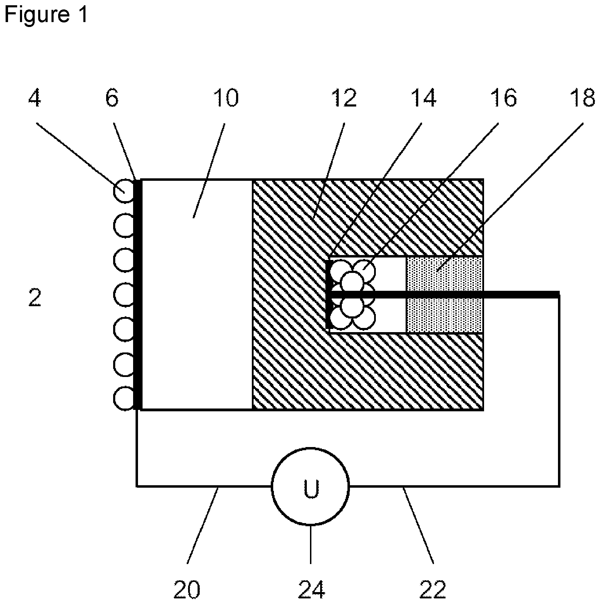

Figure 1 is a schematic cross-section of a first embodiment of the invention in the form of a galvanic cell for the sensing of molecular gases. This shows the gas 2 and, electrically connected in series, a gas-sensitive measuring electrode 4, a first electronically-conducting contact 6, a salt solid ion-conductor 10, a ceramic solid metal-ion-conductor 12, a second electronically conducting contact 14, and a reference electrode 16 that is not open to the gas. The reference electrode is contained in a blind-ended recess in the ceramic ion-conductor and is closed from the gas by a seal 18. A metal lead to the first electronically-conducting contact 20 and a metal lead to the second electronically-conducting contact 22 are connected to an electrometer or voltmeter 24 for making measurements.

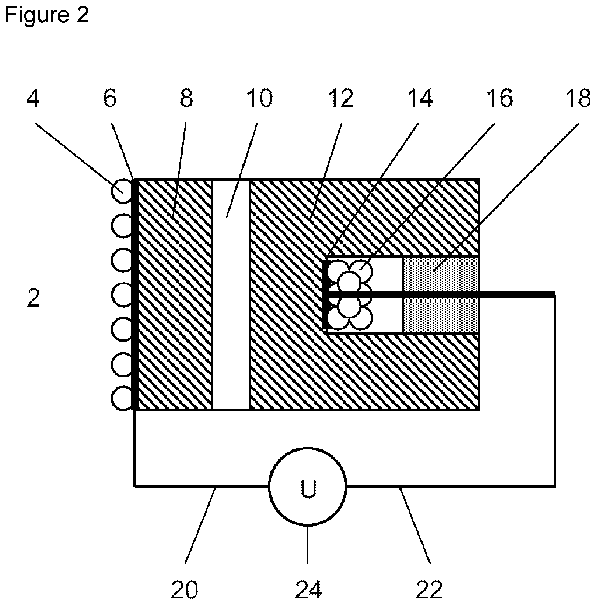

Figure 2 is a schematic cross-section of a first embodiment of the invention in the form of a galvanic cell for the sensing of molecular gases. The same reference numerals are used in Figure 2 as in Figure 1, where appropriate. Figure 1 shows the gas 2 and, electrically connected in series, a gas-sensitive measurement electrode 4, a first electronically-conducting contact 6, a first ceramic solid metal-ion-conductor 8, a salt solid ion-conductor 10, a second ceramic solid metal-ion-conductor 12, a second electronically-conducting contact 14, and a reference electrode 16 that is not open to the gas. The reference electrode is contained in a blind-ended recess in the second ion-conductor and is closed from the gas by a seal 18. A metal lead to the first electronically-conducting contact 20 and a metal lead to the second electronically-conducting contact 22 are connected to an electrometer or voltmeter 24 for making measurements.

As described below, in embodiments of the invention the salt ion-conductor 10 may be formed in various ways, including as a separate component or as a surface layer on one or both of the adjacent ceramic ion-conductors.

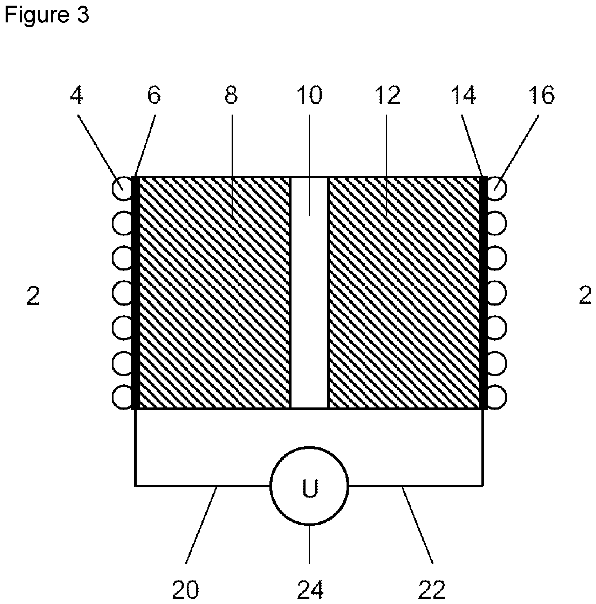

Figure 3 is a schematic cross-section of a second embodiment of the invention in the form of a novel galvanic cell for the sensing of molecular gases. This includes the gas 2 and, electrically connected in series, a gas sensitive measurement electrode 4, a first electronically-conducting contact 6, a first ceramic solid metal-ion-conductor 8, a salt solid ion-conductor 10, a second ceramic solid metal-ion-conductor 12, a second electronically-conducting contact 14, and a reference electrode 16 that is open to the gas. A metal lead to the first electronically-conducting contact 20 and a metal lead to the second electronically-conducting contact 22 are connected to an electrometer 24.

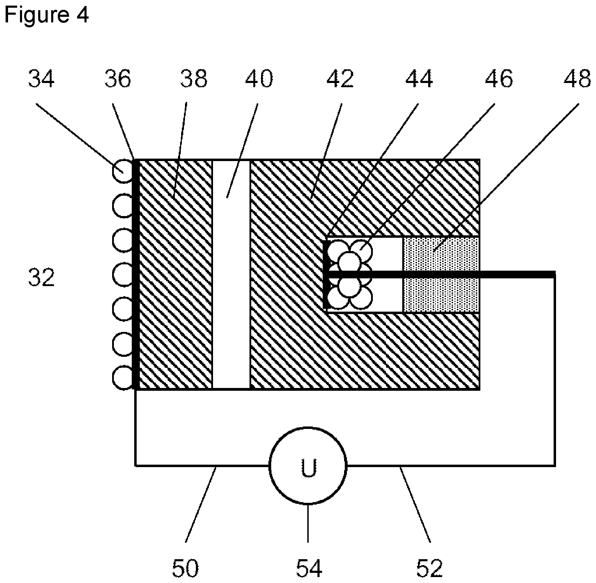

Figure 4 is a schematic cross-section of a third embodiment of the invention in the form of a galvanic cell for the sensing of $CO_2$ and $O_2$ gases. The structure of the sensor is the same as in Figure 1, but in this case the gas 32 contains $CO_2$ and $O_2$, the gas sensitive measurement electrode is $Na_2CO_3$ 34 which is exposed to the gas, and the electronically-conducting contact at the measurement electrode is platinum or gold 36. The chemical activity of Na in the measurement electrode arises from its equilibration with the $CO_2$ and $O_2$. The combination of solid ion-conductors comprises a first Na-$\beta$-alumina body 38, a $Na_2SO_4$ body 40, and a second Na-$\beta$-alumina body 42. The electronically-conducting contact at the reference electrode is platinum, gold, tungsten, steel, Inconel, a combination thereof, or some other suitable refractory electronic conductor 44, the reference electrode is pure Na metal 46 which is separated from the environment by means of a glass seal 48. The chemical activity of Na in the reference electrode is unity. The metal leads, or lead wires, coupling the electronically-conducting contacts of the two electrodes to the electrometer 54 are of platinum, gold, tungsten, steel, Inconel, a combination thereof, or some other suitable refractory electronic conductor 50, 52. The Na-$\beta$-alumina body 42 that contains the reference electrode is gastight. In other embodiments, the $Na_2SO_4$ body 40 may be replaced with a body of $Na_2CO_3$, $Na_2SiO_3$, NaF, NaCl, $CaF_2$, or some other suitable aforementioned material.

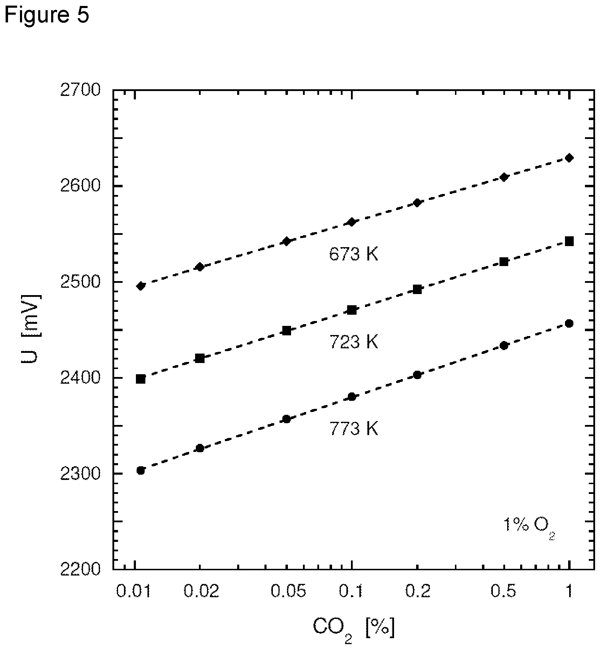

Figure 5 is a graph showing the cell potential of the cell in Figure 4 as a function of the $CO_2$ concentration of a measured gas. In experiments, the $CO_2$ concentration was varied between 0.01 and 1 % by volume, the $O_2$ concentration was fixed at 1 % by volume, the balance gas was argon, and the temperature was varied between 400 and 500 °C (673 and 773 K). The solid symbols in the graph represent measured values and the dashed lines represent thermodynamically-expected values. The cell provided potentials that coincided to within +/- 1 mV with the theoretical values, irrespective of whether a body of $Na_2SO_4$, $Na_2CO_3$, $Na_2SiO_3$, NaF, NaCl or $CaF_2$ was used in between the two Na-$\beta$-alumina bodies.

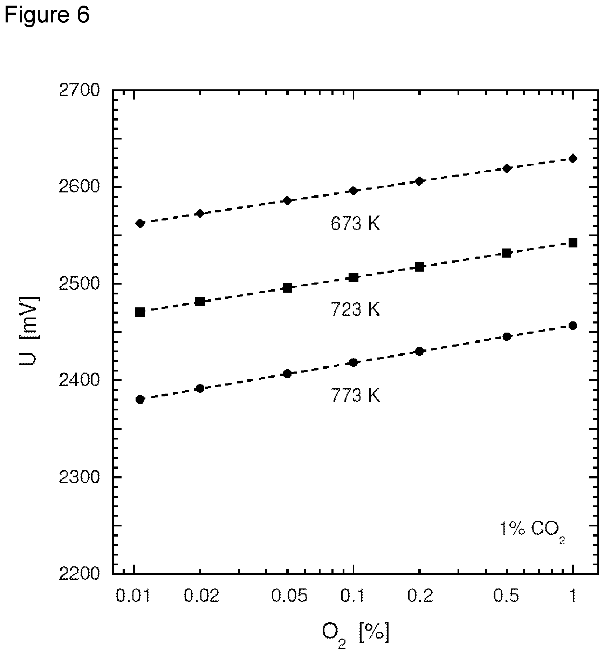

Figure 6 is a graph showing the cell potential of the cell in Figure 4 as a function of the $O_2$ concentration of the measured gas. In experiments, the $O_2$ concentration was varied between 0.01 and 1 % by volume, the $CO_2$ concentration was fixed at 1 % by volume, the balance gas was argon, and the temperature was varied between 400 and 500 °C (673 and 773 K). The solid symbols represent the measured values and the dashed lines represent the thermodynamically expected values. The cell provided potentials that coincided to within +/- 1 mV with the theoretical values, irrespective of whether a body of $Na_2SO_4$, $Na_2CO_3$, $Na_2SiO_3$, NaF, NaCl or $CaF_2$ was used in between the two Na-$\beta$-alumina bodies.

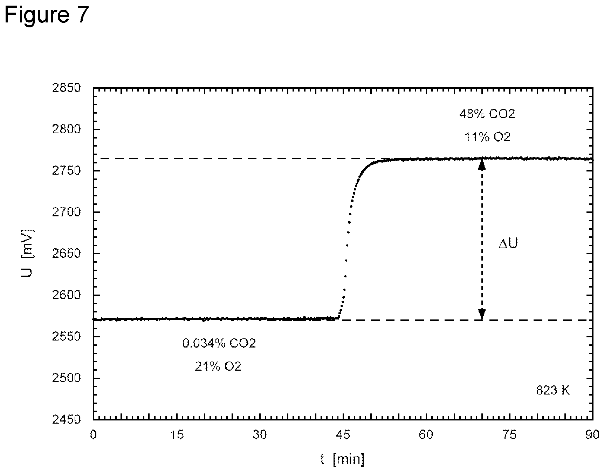

Figure 7 is a graph showing the time-dependent cell potential of the cell in Figure 4 when responding to a variation of the $CO_2$ and $O_2$ concentrations of the measured gas, using a $Na_2SO_4$ body in between the two Na-$\beta$-alumina bodies. The gas composition was changed from 0.0034 % by volume $CO_2$ and 21 % by volume $O_2$ to 45 % by volume $CO_2$ and 11 % by volume $O_2$, the balance gas was argon, and the temperature was 550 °C (823 K). The dotted curve represents the measured values and the dashed lines represent the theoretically expected values for the two compositions. The double arrow indicates the difference between the two theoretically expected values. The cell potentials measured before and after the change of composition coincided to within +/- 1 mV with the theoretical values. Accordingly, the measured cell potential difference during the response of the cell to the variation of the gas composition also corresponded to the theoretically expected one.

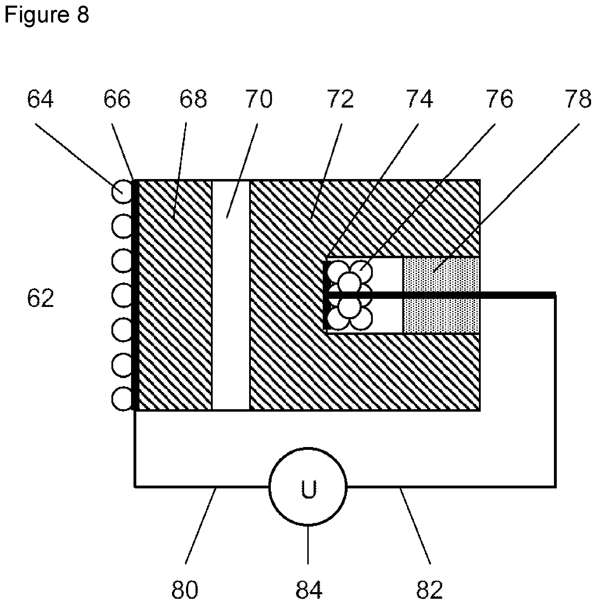

Figure 8 is a schematic cross-section of a fourth embodiment of the invention in the form of a galvanic cell for the sensing of $SO_3$ and $O_2$ gases. The structure of the sensor is similar to that of Figure 3, but in this case the gas 62 contains $SO_3$ and $O_2$, the gas sensitive measurement electrode is $Na_2SO_4$ 64 which is exposed to the gas, and the electronically-conducting contact at the measurement electrode is platinum or gold 66. The chemical activity of Na in the measurement electrode arises from its equilibration with the $SO_3$ and $O_2$. The combination of solid ion-conductors comprises a first Na-$\beta$-alumina body 68, a $Na_2SO_4$ body 70, and a second Na-$\beta$-alumina body 72. The electronically-conducting contact at the reference electrode is platinum, gold, tungsten, steel, Inconel, a combination thereof, or some other suitable refractory electronic conductor 74, the reference electrode is pure Na metal 76 which is separated from the environment by means of a glass seal 78. The chemical activity of Na in the reference electrode is unity. The metal leads, or lead wires, coupling the electronically-conducting contacts of the two electrodes to the electrometer 84 are of platinum, gold, tungsten, steel, Inconel, a combination thereof, or some other suitable refractory electronic conductor 80, 82. The Na-$\beta$-alumina body 72 that contains the reference electrode is gastight.

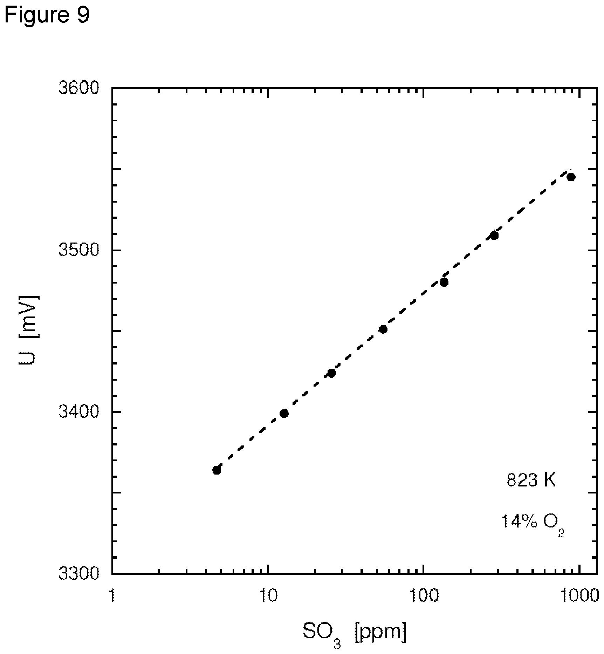

Figure 9 is a graph showing the cell potential of the cell in Figure 8 as a function of the $SO_3$ concentration of the measured gas. In experiments, the $SO_3$ concentration was varied between 5 and 900 ppm by volume, the $O_2$ concentration was fixed at 14 % by volume, the balance gas was argon, and the temperature was 550 °C (823 K). The solid symbols represent the measured values and the dashed lines represent the thermodynamically expected values. The cell provided potentials that coincided to within +/- 1 mV with the theoretical values.

In the inventors' experiments several sensors embodying the invention were assembled and tested with respect to their ability to sense molecular gases. The synthesis of the components and their assembly into a sensor are now described.

Na-$\beta$-alumina powders were prepared through a solid-state chemical route. The synthesis included drying $Na_2CO_3$ and $\gamma$-AlOOH (Boehmite) powders, mixing appropriate quantities of the powders, wet milling of the powders in distilled water using $Al_2O_3$ milling spheres, drying, sieving, calcining at 1400 °C in air for 8 h, re-grinding in distilled water and in the presence of an organic binder/plasticiser system such as PVA/PEG (poly-vinyl-alcohol/poly-ethyleneglycol), and again drying and sieving. Discs were prepared by uniaxial pressing. Open-ended tubes were prepared either by isostatic pressing in suitable rubber moulds, or by pressing rectangular bars and drilling cavities into them subsequent to sintering. Sintering of the pressed bodies was performed at temperatures of 1500 to 1600 °C in air for durations of 6 to 30 min. X-ray diffraction analysis proved the successful synthesis of both single-phase material of composition $Na_2O \cdot 11Al_2O_3$ and two-phase material composed of $Na_2O \cdot 5Al_2O_3$ and $Na_2O \cdot 11Al_2O_3$. Both materials were appropriate for use as the ceramic solid metal-ion-conductor.

In each case, a working electrode was prepared by first painting a quantity of a commercial platinum ink or gold ink onto one side of a Na-$\beta$-alumina disc. This was heated at 900 °C in ambient air for 2 h. To form a measurement electrode for $CO_2$ sensing, the platinum or gold coating formed was impregnated with a $Na_2CO_3$-containing ethanol slurry and dried at 150 °C in air for several hours. To form a measurement electrode for $SO_3$ sensing, the platinum or gold coating was impregnated with a $Na_2SO_4$-containing ethanol slurry and dried at 150 °C in air for several hours. To form a measurement electrode for $NO_2$ sensing, the platinum or gold coating was impregnated with a $NaNO_3$-containing ethanol slurry and dried at 150 °C in air for several hours. To form a measurement electrode for $Cl_2$ sensing, the platinum or gold coating was impregnated with a NaCl-containing ethanol slurry and dried at 150 °C in air for several hours.

To form a Na metal reference electrode, a quantity of typically 1 to 3 mg of pure Na metal was placed at the lower end of the inner cavity within an open-ended Na-$\beta$-alumina tube. The tube then had a hermetical seal formed at its upper end that consisted of a sealing glass resistant to Na. The sealing glass was an oxide mixture composed of, exclusively, CaO, $Al_2O_3$, BaO and $B_2O_3$. The seal was formed by placing either a quantity of glass powder or a suitably-shaped solid glass body on top of the open-ended Na-$\beta$-alumina tube, heating this arrangement to slightly above the melting temperature of the glass in an atmosphere of dry argon or other inert gas, and then cooling it down to again. Glasses composed of the above oxides have melting points as low as around 800 °C and are therefore useable in conjunction with elemental Na.

Salt ion-conductors were prepared and applied in contact with the ceramic ion-conductor in different manners to form different sensors for testing: (1) as a body of the pure material, (2) as an infiltrate within a body of a porous support structure of a different chemical composition, or (3) as a surface film on the ceramic ion-conductor.

In the first approach, discs of the salt solid ion-conductor were prepared by uniaxial pressing of powders of salts such as $Na_2SO_4$, $Na_2CO_3$, $Na_2SiO_3$, NaF, NaCl or $CaF_2$, followed by sintering at temperatures below their melting points for several hours. A typical sintering temperature was 800 °C, and a typical sintering time was 8 h.

In the second approach, refractory ceramic discs made of materials such as MgO, $Al_2O_3$, $ZrO_2$ or $Y_2O_3$ and with a significant degree of continuous open porosity, were infiltrated in vacuum with molten salts such as $Na_2SO_4$, $Na_2CO_3$, $Na_2SiO_3$, NaF, NaCl or $CaF_2$, so that after cooling a continuous path of solidified salt was present throughout the entire refractory ceramic disc.

In the third approach, the salt ion-conductor was formed through a chemical reaction between the surface of the Na-$\beta$-alumina disc or blind-ended tube (i.e. the ceramic ion-conductor) and a suitable gas, such as $CO_2$, $SO_2$ or $Cl_2$. The gas contained between a few ppm and 100 % of one or more of these components. $O_2$ may be present in the gas when $CO_2$ or $SO_2$ are used, but not when $Cl_2$ is used. Chemically inert diluent gases such as nitrogen, argon or others may be present or absent in all cases.

To form each sensor, a disc of Na-$\beta$-alumina carrying a measurement electrode and constituting the first ceramic solid metal-ion-conductor, a disc comprising $Na_2SO_4$, $Na_2CO_3$, $Na_2SiO_3$, NaF, NaCl or $CaF_2$ constituting the salt solid ion-conductor, and a blind-ended tube of Na-$\beta$-alumina containing a Na metal reference electrode and constituting the second ceramic solid metal-ion-conductor, were brought into contact with each other, in this order, in a custom-built quartz jig. (In the third approach described above, the disc comprising the salt ion-conductor is the layer formed on the surface of the Na-β-alumina disc or blind-ended tube.) As indicated in the figures, the measurement electrode faced away from the series arrangement of the three solid ion-conductors and for exposure, in use, to the gas to be analysed, and the reference electrode faced away from the series arrangement of the three solid ion-conductors in the opposite direction and was protected from the gas by a seal.

Experiments for the sensing of $CO_2$ and $O_2$ were carried out with a galvanic cell having the structure shown in Figure 4 that included $Na_2CO_3$ as the measurement electrode, Na-β-alumina as the ceramic solid metal-ion-conductor and $Na_2SO_4$ as the salt solid ion-conductor. Cell potentials were measured between the measurement electrode and the reference electrode with a high-impedance electrometer. Extensive long-term studies were performed with gas mixtures containing up to 1 % by volume of $CO_2$ and up to 1 % by volume of $O_2$, as it is considered that the type of sensor described is expected to have its main application in the field of trace analysis. In one set of experiments, the $CO_2$ concentration was varied between 0.01 and 1 % by volume, the $O_2$ concentration was constant at 1 % by volume, the balance gas was argon, and the temperature was between 400 and 500 °C (673 and 773 K). The cell potentials measured under these conditions were found to be identical to those calculated from published thermodynamic data to within +/- 1 mV. Figure 5 shows the results. In another set of experiments, the $O_2$ concentration was varied between 0.01 and 1 % by volume, the $CO_2$ concentration was constant at 1 % by volume, the balance gas was argon, and the temperature was between 400 and 500 °C (673 and 773 K). The cell potentials measured were again found to be identical to those calculated from published thermodynamic data to within +/- 1 mV. Figure 6 shows the results.

In an alternative arrangement, experiments for the sensing of $CO_2$ and $O_2$ were carried out with a galvanic cell having the structure shown in Figure 4 that included $Na_2CO_3$ as the salt solid ion-conductor. Other conditions were as described above. Identical cell potentials were measured.

In a further alternative arrangement, experiments for the sensing of $CO_2$ and $O_2$ were carried out with a galvanic cell having the structure shown in Figure 4 that included $Na_2SiO_3$ as the salt solid ion-conductor. Other conditions were as described above. Identical cell potentials were measured.

In yet a further alternative arrangement, experiments for the sensing of $CO_2$ and $O_2$ were carried out with a galvanic cell having the structure shown in Figure 4 that included $CaF_2$ as the salt solid ion-conductor. Other conditions were as described above. Identical cell potentials were measured.

The time dependence of the sensor response to variations in the composition of the gas was investigated with two gases of very different $CO_2$ concentrations, so as to achieve a quantitatively larger change in cell potential. In the experiment, response behaviour was measured by quickly changing the composition of the gas from 0.0034 % by volume $CO_2$ and 21 % by volume $O_2$ to 45 % by volume $CO_2$ and 11 % by volume $O_2$ (balance gas argon) at the temperature of 550 °C (823 K) and following the cell potential as a function of time. The response was fast with 90 % of the total cell potential change achieved within less than 5 min. Figure 7 shows a typical result.

The stability of the cell potential over time was ascertained by measuring the cell potential as a function of time at a constant gas composition and a constant temperature. Cell potentials remained constant under these conditions to within +/- 1 mV over extended periods of time, with six weeks being the longest single measurement performed. There was no indication that longer measuring times would lead to cell-potential drift.

Experiments found that the preferred temperature range for gas sensing with the above type of galvanic cell is between 300 and 600 °C. Temperatures lower than 300 °C are less preferred, because these led to deviations between the measured and the thermodynamically expected cell potential, probably because of an increase in cell impedance. Temperatures higher than 600 °C are less preferred, because of their adverse impact on the sensor durability.

Experiments for the sensing of $SO_3$ and $O_2$ were carried out with a galvanic cell having the structure shown in Figure 8 that included $Na_2SO_4$ as the measurement electrode, Na-β-alumina as the ceramic solid metal-ion-conductor and $Na_2SO_4$ as the salt solid ion-conductor. Cell potentials were measured between the measurement electrode and the reference electrode with a high-impedance electrometer. In the experiment, the $SO_3$ concentration was varied between 5 and 900 ppm by volume, the $O_2$ concentration was constant at 14 % by volume, the balance gas was argon, and the temperature was 550 °C (823 K). The cell potentials measured under these conditions were found to be identical to those calculated from published thermodynamic data to within +/- 4 mV. Figure 9 shows the results.

Control experiments were performed with galvanic cells of the above type but without the salt solid ion-conductor in between the two ceramic solid metal-ion-conductors, and these yielded measured cell potentials that were, without exception, lower by several hundreds of millivolts than the thermodynamically-expected values. A clear response behaviour to changes in the concentration of the target species in the gas was detected, but the response was never fully quantitative and was typically followed by significant drift.

It is understood that the measurements described above have been obtained with specific embodiments of the present invention. Various other geometric arrangements and materials combinations may be realised without departing from the scope of the present invention, which is solely defined by the appended claims. A few of these embodiments are now named. All sensor components may be used in arbitrary geometries other than those mentioned. A combination of more than three solid ion-conductors may be used. Ceramic solid metal-ion-conductors other than Na-β-alumina may be used, most importantly NASICON, but also many others including those mentioned afore. Salt solid ion-conductors

other than $Na_2SO_4$, $Na_2CO_3$, $Na_2SiO_3$, NaF, NaCl or $CaF_2$ may be used. The salt solid ion-conductors may not only be used in their pure forms but also in mixtures or in their partially-substituted forms; for example, $Na_2SO_4$ may contain La and/or Y as a partial substitute for Na, and $Na_2SO_4$ may contain $WO_4^{2-}$ ions as a partial substitute for $SO_4^{2-}$. Both the ceramic solid ion-conductor and the salt solid ion-conductor may contain admixtures of non-conducting second phases, such as $ZrO_2$, $Y_2O_3$, $SiO_2$ or others. Reference electrodes other than pure Na metal may be used, for example, alloys, intermetallics or compounds containing Na, either protected from the gas by a seal or open to the gas.

**Claims**

1. A sensor for a molecular gas, comprising:

   a gas-sensitive measurement electrode (4, 6, 34, 36, 64, 66),
   a reference electrode (14, 16, 44, 46, 74, 76), and
   an electrolyte (8, 10, 12, 38, 40, 42, 68, 70, 72) in electrical contact with the measurement electrode and the reference electrode,
   **characterised in that** the electrolyte comprises, arranged in series between the measurement electrode and the reference electrode:

   a first ion-conductor (8, 12, 38, 42, 68, 72), which is a ceramic or glass solid ion-conductor, and
   a second ion-conductor (10, 40, 70), which comprises an ion-conducting salt.

2. A sensor according to claim 1, in which the second ion-conductor is a solid ion-conductor, and preferably comprises a solid body.

3. A sensor according to claim 1 or 2, in which the measurement electrode comprises an auxiliary phase (6, 36, 66) exposable, in use, to a measurand gas comprising the molecular gas, and an electronic contact (4, 34, 64) in electrical contact with the auxiliary phase.

4. A sensor according to any preceding claim, in which the second ion-conductor comprises a salt containing one or more cations selected from the group consisting of lithium, sodium, potassium, rubidium, caesium, magnesium, calcium, strontium, barium, lanthanum, yttrium and other rare-earth metals;
   and/or containing one or more anions selected from the group consisting of carbonate, nitrate, sulphate, meta-silicate, ortho-silicate, ortho-phosphate, fluoride, chloride and bromide;
   for example comprising one or more materials selected from the group comprising $Na_2CO_3$, $Na_2SO_4$, $Na_2SiO_3$, NaF, NaCl, $CaF_2$, a mixture or combination of these materials, and any of these materials in partially-substituted form.

5. A sensor according to any preceding claim, in which the second ion-conductor comprises an infiltrate within a porous support structure, the porous support structure preferably comprising a refractory ceramic material, such as MgO, $Al_2O_3$, $ZrO_2$ or $Y_2O_3$.

6. A sensor according to any preceding claim, in which the electrolyte comprises a third ion-conductor (8, 38, 68), which is ceramic or glass solid ion-conductor;
   in which, preferably, the second ion-conductor is in contact with the third ion-conductor and separates the first and third ion-conductors;
   and in which, particularly preferably, the first ion-conductor has the first ion as its mobile species, the second ion-conductor has the second ion as its mobile species, and the third ion-conductor has a third ion as its mobile species, in which the first, second and third ions are all different ions, or two of the first, second and third ions are the same ions, or all of the first, second and third ions are the same ions.

7. A sensor according to any preceding claim, in which the first ion-conductor is a metal-ion-conductor, and preferably an alkali-metal ion, alkaline-earth-metal ion, copper ion, silver ion or rare-earth-metal ion conductor, or is a sodium ion-conductor;
   or in which the first ion-conductor comprises Na-β-alumina or NASICON, or comprises one or more materials selected from the group consisting of Li-β-alumina, Na-β-alumina, K-β-alumina, Mg-β-alumina, Ca-β-alumina, Sr-β-alumina, Ba-β-alumina, Cu-β-alumina, Ag-β-alumina, rare earth metal β-aluminas, LISICON, CuSiCON and oxide glass.

8. A sensor according to claim 6, in which the third ion-conductor is a metal-ion-conductor, and preferably an alkali-metal ion, alkaline-earth-metal ion, copper ion, silver ion or rare-earth-metal ion conductor, or is a sodium ion-conductor;

or in which the third ion-conductor comprises Na-$\beta$-alumina or NASICON, or comprises one or more materials selected from the group consisting of Li-$\beta$-alumina, Na-$\beta$-alumina, K-$\beta$-alumina, Mg-$\beta$-alumina, Ca-$\beta$-alumina, Sr-$\beta$-alumina, Ba-$\beta$-alumina, Cu-$\beta$-alumina, Ag-$\beta$-alumina, rare earth metal $\beta$-aluminas, LISICON, CuSiCON and oxide glass.

9. A sensor according to any preceding claim, in which the second ion-conductor comprises a surface modification of the first ion-conductor, preferably formed by exposing the surface to a gas;

for example in which the second ion-conductor comprises one or more materials selected from the group consisting of $Na_2CO_3$, $Na_2SO_4$ and NaCl, preferably prepared by exposing the first ion-conductor to a gas containing one or more materials selected from the group consisting of $CO_2$, $SO_2$, $Cl_2$, $O_2$ and inert gases.

10. A sensor according to claim 6 or 8, in which the second ion-conductor comprises a surface modification of the third ion-conductor, preferably formed by exposing the surface to a gas;

for example in which the second ion-conductor comprises one or more materials selected from the group consisting of $Na_2CO_3$, $Na_2SO_4$ and NaCl, preferably prepared by exposing the third ion-conductor to a gas containing one or more materials selected from the group consisting of $CO_2$, $SO_2$, $Cl_2$, $O_2$ and inert gases.

11. A sensor according to any preceding claim, in which the reference electrode comprises a unary, binary or ternary compound, or mixture of such compounds, that provides a predetermined chemical activity of the metal whose ion is mobile in the second ion-conductor;

in which the metal preferably comprises Na, particularly preferably elemental Na.

12. A sensor according to any preceding claim, in which the electrolyte comprises more than two ceramic or glass solid ion-conductors arranged in series between the measurement electrode and the reference electrode;

or in which the electrolyte comprises more than one ion-conductor comprising a salt, arranged in series between the measurement electrode and the reference electrode, the or each ion-conductor being spaced from each of the measurement electrode and the reference electrode by one of the ceramic or glass solid ion-conductors.

13. A method for sensing a molecular gas, comprising the steps of:

exposing a gas-sensitive measurement electrode (4, 6, 34, 36, 64, 66) to a measurand gas (2, 32, 62) comprising the molecular gas,
generating a reference potential at a reference electrode (14, 16, 44, 46, 74, 76),
allowing ionic conduction through an electrolyte (8, 10, 12, 38, 40, 42, 68, 70, 72) between the measurement electrode and the reference electrode, and
measuring a potential difference (24, 54, 84) between the measurement electrode and the reference electrode, **characterised by** the electrolyte comprising a first ion-conductor (8, 12, 38, 42, 68, 72), which is a ceramic or glass solid ion-conductor, and a second ion-conductor (10, 40, 70), which comprises an ion-conducting salt, arranged in series between the measurement electrode and the reference electrode.

14. A method for making a sensor for sensing a molecular gas, comprising the steps of:

arranging an electrolyte (8, 10, 12, 38, 40, 42, 68, 70, 72) between a gas-sensitive measurement electrode (4, 6, 34, 36, 64, 66) and a reference electrode (14, 16, 44, 46, 74, 76),
**characterised in that** the electrolyte comprises a first ion-conductor (8, 12, 38, 42, 68, 72), which is a ceramic or glass solid ion-conductor, and a second ion-conductor (10, 40, 70), which comprises an ion-conducting salt, arranged in series between the measurement electrode and the reference electrode;
optionally further comprising the step of forming the second conductor as a surface modification of the first ion-conductor, preferably by exposing a surface of the first ion-conductor to a gas.

15. A method according to claim 14, comprising the step of additionally arranging a third ion-conductor (8, 38, 68), which is ceramic or glass solid ion-conductor, arranged in series between the measurement electrode and the reference electrode;

preferably comprising the step of forming the second ion-conductor as a surface modification of the third ion-conductor, particularly preferably by exposing a surface of the third ion-conductor to a gas.

**Patentansprüche**

1. Sensor für ein molekulares Gas, der Folgendes aufweist:

   eine gasempfindliche Messelektrode (4, 6, 34, 36, 64, 66),
   eine Referenzelektrode (14, 16, 44, 46, 74, 76) und
   einen Elektrolyt (8, 10, 12, 38, 40, 42, 68, 70, 72), der mit der Messelektrode und der Referenzelektrode in elektrischem Kontakt ist,
   **dadurch gekennzeichnet, dass** der Elektrolyt Folgendes zwischen der Messelektrode und der Referenzelektrode in Reihe angeordnet aufweist:

   einen ersten Ionenleiter (8, 12, 38, 42, 68, 72), der ein fester Keramik- oder Glas-Ionenleiter ist, und
   einen zweiten Ionenleiter (10, 40, 70), der ein ionenleitendes Salz aufweist.

2. Sensor nach Anspruch 1, bei dem der zweite Ionenleiter ein fester Ionenleiter ist und vorzugsweise einen festen Körper aufweist.

3. Sensor nach Anspruch 1 oder 2, bei dem die Messelektrode eine Hilfsphase (6, 36, 66), die einem das molekulare Gas aufweisenden Messgas ausgesetzt werden kann, und einen elektronischen Kontakt (4, 34, 64), der mit der Hilfsphase in elektrischem Kontakt ist, aufweist.

4. Sensor nach einem der vorhergehenden Ansprüche, bei dem der zweite Ionenleiter ein Salz aufweist, das ein oder mehr Kationen enthält, die aus der Gruppe bestehend aus Lithium, Natrium, Kalium, Rubidium, Caesium, Magnesium, Calcium, Strontium, Barium, Lanthan, Yttrium und anderen Seltenerdmetallen ausgewählt sind; und/oder ein oder mehr Anionen enthält, die aus der Gruppe bestehend aus Carbonat, Nitrat, Sulfat, Metasilikat, Orthosilikat, Orthophosphat, Fluorid, Chlorid und Bromid ausgewählt sind; wobei er zum Beispiel ein oder mehr Materialien aufweist, die aus der Gruppe, die $Na_2CO_3$, $Na_2SO_4$, $Na_2SiO_3$, $NaF$, $NaCl$, $CaF_2$ aufweist, einem Gemisch oder einer Kombination aus diesen Materialien und irgendwelchen dieser Materialien in teilsubstituierter Form ausgewählt sind.

5. Sensor nach einem der vorhergehenden Ansprüche, bei dem der zweite Ionenleiter ein Infiltrat in einer porösen Trägerstruktur aufweist, wobei die poröse Trägerstruktur vorzugsweise ein feuerfestes Keramikmaterial, wie etwa $MgO$, $Al_2O_3$, $ZrO_2$ oder $Y_2O_3$, aufweist.

6. Sensor nach einem der vorhergehenden Ansprüche, bei dem der Elektrolyt einen dritten Ionenleiter (8, 38, 68) aufweist, der ein fester Keramik- oder Glas-Ionenleiter ist; bei dem vorzugsweise der zweite Ionenleiter mit dem dritten Ionenleiter in Kontakt ist und den ersten und den dritten Ionenleiter voneinander trennt; und bei dem, besonders vorzugsweise, der erste Ionenleiter das erste Ion als seine mobile Spezies hat, der zweite Ionenleiter das zweite Ion als seine mobile Spezies hat und der dritte Ionenleiter das dritte Ion als seine mobile Spezies hat, bei dem das erste, zweite und dritte Ion alle verschiedene Ionen sind oder zwei von dem ersten, zweiten und dritten Ion die gleichen Ionen sind oder alle von dem ersten, zweiten und dritten Ion die gleichen Ionen sind.

7. Sensor nach einem der vorhergehenden Ansprüche, bei dem der erste Ionenleiter ein Metallionenleiter und vorzugsweise ein Alkalimetallionen-, Erdalkalimetallionen-, Kupferionen-, Silberionen- oder Seltenerdmetallionenleiter ist oder ein Natriumionenleiter ist; oder bei dem der erste Ionenleiter Na-β-Tonerde oder NASICON aufweist oder ein oder mehr Materialien aufweist, die aus der Gruppe bestehend aus Li-β-Tonerde, Na-β-Tonerde, K-β-Tonerde, Mg-β-Tonerde, Ca-β-Tonerde, Sr-β-Tonerde, Ba-β-Tonerde, Cu-β-Tonerde, Ag-β-Tonerde, Seltenerdmetall-β-Tonerden, LISICON, CuSiCON und Oxidglas ausgewählt sind.

8. Sensor nach Anspruch 6, bei dem der dritte Ionenleiter ein Metallionenleiter und vorzugsweise ein Alkalimetallionen-, Erdalkalimetallionen-, Kupferionen-, Silberionen- oder Seltenerdmetallionenleiter ist oder ein Natriumionenleiter ist; oder bei dem der dritte Ionenleiter Na-β-Tonerde oder NASICON aufweist oder ein oder mehr Materialien aufweist, die aus der Gruppe bestehend aus Li-β-Tonerde, Na-β-Tonerde, K-β-Tonerde, Mg-β-Tonerde, Ca-β-Tonerde, Sr-β-Tonerde, Ba-β-Tonerde, Cu-β-Tonerde, Ag-β-Tonerde, Seltenerdmetall-β-Tonerden, LISICON, CuSiCON und Oxidglas ausgewählt sind.

9. Sensor nach einem der vorhergehenden Ansprüche, bei dem der zweite Ionenleiter eine Oberflächenmodifikation des ersten Ionenleiters aufweist, die vorzugsweise gebildet wird, indem die Oberfläche Gas ausgesetzt wird; bei dem zum Beispiel der zweite Ionenleiter ein oder mehr Materialien aufweist, die aus der Gruppe, die aus $Na_2CO_3$, $Na_2SO_4$ und NaCl besteht, ausgewählt sind, die vorzugsweise dadurch hergestellt werden, dass der erste Ionenleiter einem Gas ausgesetzt wird, das ein oder mehr Materialien enthält, die aus der Gruppe ausgewählt sind, die aus $CO_2$, $SO_2$, $Cl_2$, $O_2$ und Inertgasen besteht.

10. Sensor nach Anspruch 6 oder 8, bei dem der zweite Ionenleiter eine Oberflächenmodifikation des dritten Ionenleiters aufweist, die vorzugsweise dadurch gebildet wird, dass die Oberfläche einem Gas ausgesetzt wird; wobei der zweite Ionenleiter zum Beispiel ein oder mehr Materialien aufweist, die aus der Gruppe ausgewählt sind, die aus $Na_2CO_3$, $Na_2SO_4$ und NaCl besteht, die vorzugsweise dadurch hergestellt werden, dass der dritte Ionenleiter einem Gas ausgesetzt wird, das ein oder mehr Materialien enthält, die aus der Gruppe ausgewählt sind, die aus $CO_2$, $SO_2$, $Cl_2$, $O_2$ und Inertgasen besteht.

11. Sensor nach einem der vorhergehenden Ansprüche, bei dem die Referenzelektrode eine unäre, binäre oder ternäre Verbindung oder ein Gemisch von derartigen Verbindungen aufweist, die bzw. das eine vorbestimmte chemische Aktivität des Metalls bereitstellt, dessen Ion im zweiten Ionenleiter mobil ist; bei dem das Metall vorzugsweise Na, besonders vorzugsweise elementares Na, aufweist.

12. Sensor nach einem der vorhergehenden Ansprüche, bei dem der Elektrolyt mehr als zwei feste Keramik- oder Glas-Ionenleiter aufweist, die zwischen der Messelektrode und der Referenzelektrode in Reihe angeordnet sind; oder bei dem der Elektrolyt mehr als einen Salz aufweisenden Ionenleiter aufweist, der zwischen der Messelektrode und der Referenzelektrode in Reihe angeordnet ist, wobei der oder jeder Ionenleiter durch einen der festen Keramik- oder Glas-Ionenleiter von jeder der Messelektrode und der Referenzelektrode beabstandet ist.

13. Verfahren zum Messen eines molekularen Gases, das die folgenden Schritte aufweist:

   Aussetzen einer gasempfindlichen Messelektrode (4, 6, 34, 36, 64, 66) einem Messgas (2, 32, 62), welches das molekulare Gas aufweist;
   Erzeugen eines Referenzpotentials an einer Referenzelektrode (14, 16, 44, 46, 74, 76),
   Zulassen von Ionenleitung durch einen Elektrolyt (8, 10, 12, 38, 40, 42, 68, 70, 72) zwischen der Messelektrode und der Referenzelektrode, und
   Messen einer Potentialdifferenz (24, 54, 84) zwischen der Messelektrode und der Referenzelektrode,
   **dadurch gekennzeichnet, dass** der Elektrolyt zwischen der Messelektrode und der Referenzelektrode in Reihe angeordnet einen ersten Ionenleiter (8, 12, 38, 42, 68, 72), der ein fester Keramik- oder Glas-Ionenleiter ist, und einen zweiten Ionenleiter (10, 40, 70), der ein ionenleitendes Salz aufweist, aufweist.

14. Verfahren zum Anfertigen eines Sensors zum Messen eines molekularen Gases, das die folgenden Schritte aufweist:

   Anordnen eines Elektrolyten (8, 10, 12, 38, 40, 42, 68, 70, 72) zwischen einer gasempfindlichen Messelektrode (4, 6, 34, 36, 64, 66) und einer Referenzelektrode (14, 16, 44, 46, 74, 76),
   **dadurch gekennzeichnet, dass** der Elektrolyt zwischen der Messelektrode und der Referenzelektrode in Reihe angeordnet einen ersten Ionenleiter (8, 12, 38, 42, 68, 72), der ein fester Keramik- oder Glas-Ionenleiter ist, und einen zweiten Ionenleiter (10, 40, 70), der ein ionenleitendes Salz aufweist, aufweist;
   das wahlweise ferner den Schritt des Bildens des zweiten Leiters als eine Oberflächenmodifikation des ersten Ionenleiters, vorzugsweise dadurch, dass eine Oberfläche des ersten Ionenleiters einem Gas ausgesetzt wird, aufweist.

15. Verfahren nach Anspruch 14, das den Schritt des zusätzlichen Anordnens eines dritten Ionenleiters (8, 38, 68) aufweist, der ein zwischen der Messelektrode und der Referenzelektrode in Reihe angeordneter fester Keramik- oder Glas-Ionenleiter ist; das vorzugsweise den Schritt des Bildens des zweiten Ionenleiters als eine Oberflächenmodifikation des dritten Ionenleiters, besonders vorzugsweise dadurch, dass eine Oberfläche des dritten Ionenleiters einem Gas ausgesetzt wird, aufweist.

**Revendications**

1.  Capteur de gaz moléculaire, comprenant :

    une électrode de mesure sensible aux gaz (4, 6, 34, 36, 64, 66),
    une électrode de référence (14, 16, 44, 46, 74, 76), et
    un électrolyte (8, 10, 12, 38, 40, 42, 68, 70, 72) en contact électrique avec l'électrode de mesure et l'électrode de référence,
    **caractérisé en ce que** l'électrolyte comprend, agencés en série entre l'électrode de mesure et l'électrode de référence :

    un premier conducteur ionique (8, 12, 38, 42, 68, 72), lequel est un conducteur ionique solide en céramique ou verre, et
    un deuxième conducteur ionique (10, 40, 70), lequel comprend un sel conducteur d'ions.

2.  Capteur selon la revendication 1, dans lequel le deuxième conducteur ionique est un conducteur ionique solide, et comprend de préférence un corps solide.

3.  Capteur selon la revendication 1 ou 2, dans lequel l'électrode de mesure comprend une phase auxiliaire (6, 36, 66) pouvant être exposée, durant l'utilisation, à un gaz de mesurande comprenant le gaz moléculaire, et un contact électronique (4, 34, 64) en contact électrique avec la phase auxiliaire.

4.  Capteur selon n'importe quelle revendication précédente, dans lequel le deuxième conducteur ionique comprend un sel contenant un ou plusieurs cations sélectionnés dans le groupe consistant en lithium, sodium, potassium, rubidium, caesium, magnésium, calcium, strontium, baryum, lanthane, yttrium et autre métaux de terres rares ; et/ou contenant un ou plusieurs anions sélectionnés dans le groupe consistant en nitrate, sulfate, méta-silicate, ortho-silicate, ortho-phosphate, fluor, chlorure et bromure ; par exemple comprenant un ou plusieurs matériaux sélectionnés dans le groupe comprenant $Na_2CO_3$, $NaSO_4$, $NaSiO_3$, $NaF$, $NaCl$, $CaF_2$, un mélange ou une combinaison de ces matériaux, et n'importe lequel de ces matériaux sous forme partiellement substituée.

5.  Capteur selon n'importe quelle revendication précédente, dans lequel le second conducteur ionique comprend un infiltrat dans une structure de support poreuse, la structure de support poreuse comprenant de préférence un matériau céramique réfractaire, tel que $MgO$, $Al_2O_3$, $ZrO_2$ ou $Y_2O_3$.

6.  Capteur selon n'importe quelle revendication précédente, dans lequel l'électrolyte comprend un troisième conducteur ionique (8, 38, 68), lequel est un conducteur ionique solide en céramique ou verre ; dans lequel, de préférence, le deuxième conducteur ionique est en contact électrique avec le troisième conducteur ionique et sépare les premier et troisième conducteurs ioniques ; et dans lequel, particulièrement de préférence, le premier conducteur ionique comporte le premier ion comme son espèce mobile, le deuxième conducteur ionique comporte le deuxième ion comme son espèce mobile, et le troisième conducteur ionique comporte un troisième ion comme son espèce mobile, dans lequel les premier, deuxième et troisième ions sont tous des ions différents ou deux des premier, deuxième et troisième ions sont les mêmes ions, ou la totalité des premier, deuxième et troisième ions sont les mêmes ions.

7.  Capteur selon n'importe quelle revendication précédente, dans lequel le premier conducteur ionique est un conducteur d'ions métalliques, et de préférence un conducteur d'ions de métaux alcalins, d'ions de métaux alcalino-terreux, d'ions de cuivre, d'ions d'argent ou d'ions de métaux de terres rares, ou est un conducteur d'ions de sodium ; ou dans lequel le premier conducteur ionique comprend β-alumine de Na ou NASICON, ou comprend un ou plusieurs matériaux sélectionnés dans le groupe consistant en β-alumine de Li, β-alumine de Na, β-alumine de K, β-alumine de Mg, β-alumine de Ca, β-alumine de Sr, β-alumine de Ba, β-alumine de Cu, β-alumine d'Ag, β-alumines de métaux de terres rares, LISICON, CuSiCON et verre d'oxyde.

8.  Capteur selon la revendication 6, dans lequel le troisième conducteur ionique est un conducteur d'ions métalliques, et de préférence un conducteur d'ions de métaux alcalins, d'ions de métaux alcalino-terreux, d'ions de cuivre, d'ions d'argent ou d'ions de métaux de terres rares, ou est un conducteur d'ions de sodium ; ou dans lequel le troisième conducteur ionique comprend β-alumine de Na ou NASICON, ou comprend un ou plusieurs matériaux sélectionnés dans le groupe consistant en β-alumine de Li, β-alumine de Na, β-alumine de K, β-alumine de Mg, β-alumine de Ca, β-alumine de Sr, β-alumine de Ba, β-alumine de Cu, β-alumine d'Ag, β-alumines de métaux de terres rares, LISICON, CuSiCON et verre d'oxyde.

9. Capteur selon n'importe quelle revendication précédente, dans lequel le deuxième conducteur ionique comprend une modification de surface du premier conducteur ionique, de préférence formée en exposant la surface à un gaz ; par exemple dans lequel le deuxième conducteur ionique comprend un ou plusieurs matériaux sélectionnés dans le groupe consistant en $Na_2CO_3$, $NaSO_4$ et $NaCl$, préparé de préférence en exposant le premier conducteur ionique à un gaz contenant un ou plusieurs matériaux sélectionnés dans le groupe consistant en $CO_2$, $SO_2$, $Cl_2$, $O_2$ et des gaz inertes.

10. Capteur selon la revendication 6 ou 8, dans lequel le deuxième conducteur ionique comprend une modification de surface du troisième conducteur ionique, formée de préférence en exposant la surface à un gaz ; par exemple dans lequel le deuxième conducteur ionique comprend un ou plusieurs matériaux sélectionnés dans le groupe consistant en $Na_2CO_3$, $NaSO_4$ et $NaCl$, préparé de préférence en exposant le troisième conducteur ionique a un gaz contenant un ou plusieurs matériaux sélectionnés dans le groupe consistant en $CO_2$, $SO_2$, $Cl_2$, $O_2$ et des gaz inertes.

11. Capteur selon n'importe quelle revendication précédente, dans lequel l'électrode de référence comprend un composé unaire, binaire ou ternaire ou un mélange de tels composés, qui fournit une activité chimique prédéterminée du métal dont l'ion est mobile dans le deuxième conducteur ionique ; dans lequel le métal comprend de préférence du Na, particulièrement de préférence du Na élémentaire.

12. Capteur selon n'importe quelle revendication précédente, dans lequel l'électrolyte comprend plus de deux conducteurs ioniques solides en céramique ou verre agencés en série entre l'électrode de mesure et l'électrode de référence ; ou dans lequel l'électrolyte comprend plus d'un conducteur ionique comprenant un sel, agencé en série entre l'électrode de mesure et l'électrode de référence, le ou chaque conducteur ionique étant espacé de chacune de l'électrode de mesure et de l'électrode de référence par un des conducteurs ioniques solides en céramique ou verre.

13. Procédé de détection d'un gaz moléculaire, comprenant les étapes suivantes :

l'exposition d'une électrode de mesure sensible aux gaz (4, 6, 34, 36, 64, 66) à un gaz de mesurande (2, 32, 62) comprenant le gaz moléculaire,
la génération d'un potentiel de référence au niveau d'une électrode de référence (14, 16, 44, 46, 74, 76),
la conduction ionique libre à travers un électrolyte (8, 10, 12, 38, 40, 42, 68, 70, 72) entre l'électrode de mesure et l'électrode de référence, et
la mesure d'une différence de potentiel (24, 54, 84) entre l'électrode de mesure et l'électrode de référence, **caractérisé en ce que** l'électrolyte comprend un premier conducteur ionique (8, 12, 38, 42, 68, 72), qui est un conducteur ionique solide en céramique ou verre, et un deuxième conducteur ionique (10, 40, 70), qui comprend un sel conducteur d'ions, agencé en série entre l'électrode de mesure et l'électrode de référence.

14. Procédé de réalisation d'un capteur pour détecter un gaz moléculaire, comprenant les étapes suivantes :

la disposition d'un électrolyte (8, 10, 12, 38, 40, 42, 68, 70, 72) entre une électrode de mesure sensible aux gaz (4, 6, 34, 36, 64, 66) et une électrode de référence (14, 16, 44, 46, 74, 76),
**caractérisé en ce que** l'électrolyte comprend un premier conducteur ionique (8, 12, 38, 42, 68, 72), lequel est un conducteur ionique solide en céramique ou verre, et un deuxième ionique (10, 40, 70), lequel comprend un sel conducteur d'ions, agencés en série entre l'électro de mesure et électrodes de référence ; facultativement comprenant en outre l'étape de formation du deuxième conducteur en tant que modification de surface du premier conducteur ionique, de préférence en exposant une surface du premier conducteur ionique à un gaz.

15. Procédé selon la revendication 14, comprenant l'étape d'agencement supplémentaire d'un troisième conducteur ionique (8, 38, 68), lequel est un conducteur ionique solide en céramique ou verre, agencé en série entre l'électrode de mesure et l'électrode de référence , comprenant de préférence l'étape de formation du deuxième conducteur ionique en tant que modification de surface du troisième conducteur ionique, de préférence particulièrement en exposant une surface du troisième conducteur ionique à un gaz.

Figure 1

Figure 2

Figure 3

Figure 4

Figure 5

Figure 6

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5954930 A
- US 5080775 A
- JP 60003547 B

**Non-patent literature cited in the description**

- **H.-H. MÖBIUS.** Galvanic solid electrolyte cells for the measurement of CO2 concentrations. *Journal of Solid State Electrochemistry,* 2004, vol. 8, 94-109
- **J.W. FERGUS.** A review of electrolyte and electrode materials for high temperature electrochemical CO2 and SO2 gas sensors. *Sensors and Actuators B,* 2008, vol. 134, 1034-1041